# FASCICULE DE BREVET EUROPEEN

(11) **EP 0 832 645 B1**
(45) Date de publication et mention de la délivrance du brevet: **14.07.1999**
(21) Numéro de dépôt: 97402143.8
(22) Date de dépôt: 16.09.1997
(51) Int. Cl.: A61K 7/48, A61K 7/02

(54) **Composition cosmétique comprenant un copolymère particulier et utilisation dudit copolymère en cosmétique**
Kosmetische Zusammensetzung enthaltend ein bestimmtes Kopolymer und Verwendung dieses Kopolymers in der Kosmetik
Cosmetic composition comprising a particular copolymer and use of said copolymer in cosmetics

(30) Priorité: 20.09.1996 FR 9611512
(43) Date de publication de la demande: 01.04.1998
(73) Titulaire: L'OREAL, 75008 Paris (FR)
(72) Inventeur: Favre, Sophie, 94550 Chevilly-Larue (FR); Terren, Nadia, 94550 Chevilly-Larue (FR); Michelet, Jacques, 91160 Champlan (FR)
(74) Mandataire: Lhoste, Catherine

(56) Documents cités:
- WO-A-94/07463
- WO-A-95/03778
- US-A- 5 288 493

## Description

La présente invention a pour objet une composition notamment cosmétique pouvant en particulier se présenter sous forme d'une émulsion, et susceptible d'être utilisée pour le soin et/ou le maquillage de la peau humaine, des semi-muqueuses (lèvres), des muqueuses (lèvres, intérieur des paupières) et/ou des phanères.

Les compositions cosmétiques, notamment de maquillage telles que les rouges à lèvres et les fonds de teint, comprennent généralement des corps gras tels que des huiles et des cires, et une phase particulaire généralement composée de charges et de pigments. Elles peuvent ainsi se présenter, par exemple dans les cas des rouges à lèvres, sous forme de stick ou bâton ou sous forme de pâte souple. Les compositions de maquillage peuvent également comprendre de l'eau ou une phase hydrophile, et se présenter alors notamment sous forme d'émulsion huile-dans-eau, eau-dans-huile, émulsion multiple, solution ou gel aqueux, notamment lorsqu'il s'agit d'un fond de teint, de crème teintée, de crème de soin ou d'un produit solaire.

Ces compositions, lorsqu'elles sont appliquées sur la peau, les muqueuses ou les semi-muqueuses, présentent l'inconvénient de transférer. On entend par là que la composition est susceptible de se déposer, au moins en partie, sur certains supports avec lesquels elle est mise en contact, tels que, par exemple, un verre, un vêtement ou la peau.
En se déposant, ladite composition laisse une trace sur ledit support. Il s'en suit donc une persistance médiocre de la composition sur la peau ou les muqueuses, d'où la nécessité de renouveler régulièrement son application.
Par ailleurs, l'apparition de traces inacceptables sur certains vêtements et notamment sur les cols de chemisier peut écarter certaines femmes de l'utilisation de ce type de maquillage.
Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières.
Tous ces phénomènes engendrent d'une part une perte de la tenue de la composition (c'est-à-dire que la composition, en transférant ou en migrant, perd certains de ces composants et/ou perd de son homogénéité) et d'autre part un effet inesthétique que l'on souhaite bien évidemment éviter.

Depuis plusieurs années, de nombreux cosméticiens se sont intéressés aux compositions cosmétiques, notamment de rouge à lèvres ou de fond de teint 'sans transfert'. Ainsi, il a été envisagé des compositions de rouge à lèvres 'sans transfert' contenant de 1 à 70% en poids de résine liquide de silicone à motifs répétitifs silicates comportant des chaînes pendantes alkylées ou phénylées, de 10 à 98% en poids d'une huile de silicone volatile cyclique et des charges pulvérulentes. Il a également été envisagé des rouges à lèvres 'sans transfert' contenant une silicone volatile et une résine de silicone comportant une chaîne estérifiée pendante ayant au moins 12 atomes de carbone.

D'une manière générale, on sait maintenant que, si l'association d'huiles volatiles avec certains composés siliconés permet d'obtenir un résultat 'sans transfert' satisfaisant, elle présente néanmoins l'inconvénient de conduire, après évaporation des volatils, à un film dont le confort n'est pas optimal, notamment parce qu'il n'est pas possible d'ajouter d'huiles autres que siliconées dans ces compositions tout en conservant une qualité de 'sans transfert' correcte. En effet, les huiles hydrocarbonées, qui sont connues pour apporter notamment du confort à une composition cosmétique, ont comme inconvénient d'augmenter le transfert d'une telle composition.
Il subsiste donc le besoin d'une composition notamment cosmétique qui transfère peu ou pas du tout (c'est-à-dire d'une composition 'sans transfert') tout en possédant de bonnes propriétés cosmétiques, notamment des propriétés de glissant et de fraîcheur à l'application, ainsi que des qualités de douceur et de confort après maquillage.

Or, la demanderesse a mis en évidence que, de manière inattendue et surprenante, la présence d'un copolymère particulier, défini ci-après, dans une composition cosmétique permettait de limiter, voire de supprimer totalement, le transfert et/ou la migration, de ladite composition, et donc permettait d'en améliorer la tenue. La présente invention a donc pour but de proposer une composition qui permet d'obtenir un film de très bonne tenue, qui ne transfère pas et ne tache pas un support avec lequel il serait en contact.

L'invention a donc pour objet l'utilisation d'un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique dans une composition cosmétique, dermatologique, hygiénique et/ou pharmaceutique, afin de limiter, diminuer et/ou de supprimer le transfert et/ou la migration de ladite composition.

L'invention a également pour objet l'utilisation d'un tel copolymère, dans une composition cosmétique, dermatologique, hygiénique et/ou pharmaceutique, afin d'améliorer la tenue de ladite composition.

L'invention a encore pour objet l'utilisation d'un tel copolymère dans une composition cosmétique, dermatologique, hygiénique et/ou pharmaceutique, comme agent permettant de limiter, diminuer et/ou de supprimer le transfert et/ou la migration de ladite composition, et/ou comme agent permettant d'améliorer la tenue de ladite composition.

L'invention a également pour objet l'utilisation d'un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique dans une composition cosmétique, dermatologique, hygiénique et/ou pharmaceutique, pour former un film de ladite composition, ce film ne transférant et/ou ne migrant pas et/ou ayant une tenue améliorée.

Un autre objet de la présente invention est l'utilisation d'un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique dans une composition sans transfert.
Par composition sans transfert, on entend une composition qui transfère peu ou pas du tout au sens développé ci-dessus.
Un autre objet de l'invention est une composition cosmétique, dermatologique, hygiénique et/ou pharmaceutique, se présentant sous la forme de, et/ou comprenant, une émulsion huile-dans-eau, une émulsion eau-dans-huile, une émulsion multiple ou une solution multiphasée, comprenant en outre au moins un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique, et des pigments ayant une surface hydrophobe.
Un autre objet de l'invention est une composition cosmétique, dermatologique, hygiénique et/ou pharmaceutique, se présentant sous la forme de, et/ou comprenant, un gel aqueux, une solution aqueuse, hydroalcoolique ou multiphasée, comprenant en outre au moins un tel copolymère et des colorants hydrosolubles.
Encore un objet de l'invention est une composition de maquillage ou de soin sans transfert, comprenant au moins un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique.
Un autre objet de l'invention est un fond de teint sans transfert comprenant au moins un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique.

Encore un objet de l'invention est un procédé pour limiter, diminuer et/ou empêcher le transfert d'une composition cosmétique, dermatologique, hygiénique et/ou pharmaceutique, notamment une composition de maquillage ou de soin de la peau, des muqueuses, des semi-muqueuses et/ou des phanères, consistant à introduire dans ladite composition au moins un tel copolymère.
Un dernier objet de l'invention est un procédé de maquillage de la peau, des semi-muqueuses, des muqueuses et/ou des phanères consistant à appliquer sur sur celles-ci une composition cosmétique ou un fond de teint tels que définis ci-dessus.

Il est connu du document WO 95/03778 une composition pour former un masque arrachable comprenant un copolymère défini ci-dessus. Toutefois, dans WO 95/03778, ce copolymère est utilisé pour augmenter l'adhésion du masque à la peau et non pour limiter, diminuer et/ou supprimer le transfert et/ou la migration de ladite composition ou pour en améliorer sa tenue.

On a constaté que les compositions comprenant ledit polymère permettent l'obtention d'un film ayant une bonne affinité pour la peau et les muqueuses, ce qui peut se traduire par une meilleure tenue du film dans le temps et une bonne persistance de son homogénéité.
Les compositions selon l'invention sont facilement applicables sur la peau et permettent entre autre d'obtenir des produits de maquillage ayant une texture peu collante, qui restent confortables à porter tout au long de la journée.
De plus, leurs propriétés cosmétiques sont très intéressantes : elles procurent de la fraîcheur à l'application, ainsi qu'une grande douceur en final, et un maquillage unifiant et confortable.
Enfin, les compositions selon l'invention peuvent être démaquillées aisément, notamment avec des démaquillants traditionnels.

Les compositions selon l'invention trouvent notamment une application particulièrement intéressante dans le domaine du soin et/ou du maquillage de la peau, des muqueuses, des semi-muqueuses, et des phanères. On entend notamment par muqueuse, la partie interne de la paupière inférieure; parmi les semi-muqueuses, on entend plus particulièrement les lèvres du visage; par phanères, on entend les cils, sourcils, cheveux et ongles. Ainsi, l'invention trouve une application toute particulière dans le domaine des produits de soin et/ou de maquillage des lèvres du visage et de la peau, tels que les fonds de teint, les rouges à lèvres, les auto-bronzants ou les produits solaires.

Les compositions selon l'invention comprennent donc au moins un copolymère constitué d'une fraction majoritaire de monomère acide carboxylique monooléfiniquement insaturé en C₃-C₆ ou de son anhydride, et d'une fraction minoritaire de monomère ester à chaîne grasse d'acide acrylique. Ce copolymère peut être éventuellement réticulé.

Le copolymère selon l'invention peut être préparé en polymérisant une quantité prépondérante de monomère carboxylique monooléfiniquement insaturé ou de son anhydride, avec une quantité plus faible de monomère ester acrylique à chaîne grasse. La quantité de monomère carboxylique ou de son anhydride, est de préférence comprise entre 80 et 98% en poids et plus particulièrement entre 90 et 98% en poids; l'ester acrylique est de préférence présent dans des quantités comprises entre 2 et 20% en poids et plus particulièrement entre 2 et 10% en poids; les pourcentages sont calculés par rapport au poids des deux monomères.

Les monomères carboxyliques préférentiels sont choisis parmi ceux répondant à la formule :

CH₂=CR-COOH

dans laquelle R désigne hydrogène, halogène, hydroxyle, un groupe lactone, un groupe lactame, un groupe cyanogène (-CN), un groupe alkyle monovalent, un groupe aryle, un groupe alkylaryle, un groupe aralkyle ou un groupe cycloaliphatique.
Les monomères carboxyliques particulièrement préférés, sont choisis parmi l'acide acrylique, l'acide méthacrylique, l'anhydride maléique, et leurs mélanges.
Les monomères esters acryliques à chaîne grasse sont préférentiellement choisis parmi ceux répondant à la formule :

CH₂=CR¹-COOR²

dans laquelle R¹ est choisi dans le groupe formé par hydrogène, méthyle et éthyle et R² est un groupe alkyle en C₈-C₃₀, un groupe oxyalkyléne en C₈-C₃₀, un groupe carbonyloxyalkylène en C₈-C₃₀.
Les monomères esters particulièrement préférés sont ceux pour lesquels R¹ est hydrogène ou méthyle, et/ou ceux pour lesquels R² est un groupe alkyle en C₁₀-C₂₂. On peut notamment citer les acrylates et méthacrylates de décyle, de lauryle, de stéaryle, de béhényle ou de mélissyle.

Certains des copolymères selon l'invention sont notamment décrits dans la demande EP-A-0268164 et sont obtenus selon les méthodes de préparation décrites dans ce même document.
On peut citer plus particulièrement les copolymères vendus sous le nom PEMULEN par la Société GOODRICH, et notamment le copolymère acrylate/C₁₀-C₃₀-alkylacrylate tel que le produit PEMULEN TR 2.
On peut, bien évidemment, utiliser un mélange de plusieurs copolymères tels que ci-dessus définis.

Ces copolymères peuvent être présents dans les compositions selon l'invention à une concentration de 0,01 à 3% en poids par rapport au poids total de la composition, de préférence 0,02 à 0,6% en poids, et plus préférentiellement de 0,05 à 0,2% en poids.

La composition selon l'invention peut également comprendre un polymère poly(acide 2-acrylamido 2-méthylpropane sulfonique) réticulé et neutralisé à au moins 90%.
En effet, on a constaté que la présence d'un tel polymère permettait d'obtenir des propriétés cosmétiquement plus agréables, telles que de la douceur et de la facilité d'étalement, avec une gamme de viscosité des compositions finales importantes, pouvant aller de la forme d'un lait fluide à la forme d'une crème.
Ce polymère poly(acide 2-acrylamido 2-méthylpropane sulfonique) réticulé et pratiquement ou totalement neutralisé est généralement hydrosoluble ou gonflable dans l'eau. Il peut être caractérisé par le fait qu'il comprend, distribués de façon aléatoire :
a) de 90 à 99,9% en poids de motifs de formule générale (1) suivante : 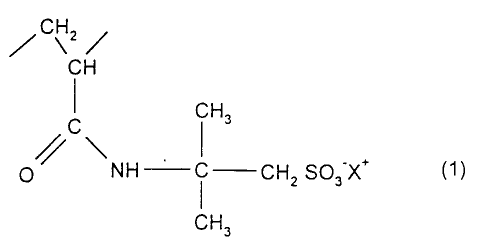 dans laquelle X⁺ désigne un cation ou un mélange de cations, au plus 10% molaire des cations X⁺ pouvant être des protons H⁺ ; et
b) de 0,01 à 10% en poids de motifs réticulants provenant d'au moins un monomère ayant au moins deux doubles liaisons oléfiniques ; les proportions en poids étant définies par rapport au poids total du polymère.
Ce polymère comprend de préférence de 98 à 99,5 % en poids de motifs de formule (1) et de 0,2 à 2 % en poids de motifs réticulants.
Le cation X⁺ représente un cation ou un mélange de cations choisis en particulier parmi un proton, un cation de métal alcalin, un cation équivalent de celui d'un métal alcalino-terreux ou l'ion ammonium. Le cation X⁺ préférentiel est le cation NH₄⁺. Plus particulièrement, 90 à 100% mole des cations sont des cations NH₄⁺ et 0 à 10% mole sont des protons (H⁺).
Les monomères de réticulation ayant au moins deux doubles liaisons oléfiniques sont choisis par exemple parmi le dipropylèneglycol-diallyléther, les polyglycoldiallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.
Les monomères de réticulation ayant au moins deux doubles liaisons oléfiniques sont plus particulièrement choisis parmi ceux répondant à la formule générale (2) suivante : 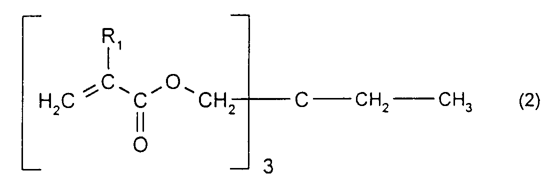 dans laquelle R₁ désigne un atome d'hydrogène ou un radical alkyle en C₁-C₄ et plus particulièrement le radical méthyle (triméthylol propane triacrylate).
Les polymères particulièrement préférés sont ceux présentant une viscosité mesurée au viscosimètre BROOKFIELD, mobile 4, à une vitesse de rotation de 100 tours/minutes, à 25°C, et en solution aqueuse à 2 % en poids, supérieure ou égale à 1000 cps (1000 mPa.s) et plus préférentiellement allant de 5000 cps à 40000 cps (5000 à 40000 mPa.s) et plus particulièrement de 6500 cps à 35000 cps.
Les poly(acide 2-acrylamido 2-méthylpropane sulfonique) réticulés, pratiquement ou totalement neutralisés peuvent être présents dans les compositions selon l'invention à une concentration de 0 à 20% en poids par rapport au poids total de la composition, de préférence 0,1 à 5% en poids, et plus préférentiellement 0,4 à 2% en poids.

Les compositions de l'invention contiennent en outre un milieu cosmétiquement, hygiéniquement, pharmaceutiquement ou dermatologiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.
Lesdites compositions contiennent un milieu aqueux cosmétiquement et/ou dermatologiquement acceptable. Dans une forme préférée de réalisation, la composition selon l'invention se présente sous la forme d'une émulsion huile-dans-eau. Toutefois, elle peut également se présenter sous la forme d'une émulsion eau-dans-huile, d'une émulsion multiple, d'un gel aqueux, ou d'une solution aqueuse, hydroalcoolique ou multiphasée, notamment eau/poudre et eau/huile/poudres.

La phase aqueuse de la composition selon l'invention peut comprendre de l'eau, une eau florale telle que l'eau de bleuet et/ou une eau minérale telle que l'eau de VITTEL, l'eau de LUCAS ou l'eau de LA ROCHE POSAY et/ou une eau thermale. Ladite phase aqueuse peut être présente à une teneur de 15 à 99,9 % en poids par rapport au poids total de la composition, de préférence 40 à 90% en poids lorsque la composition se présente sous forme d'émulsion huile-dans-eau, ou de préférence 85 à 95% en poids lorsque la composition se présente sous la forme d'un gel ou d'une solution aqueuse.
En outre, la phase aqueuse peut comprendre de 0 % à 14 % en poids, par rapport au poids total de la phase aqueuse, d'un monoalcool inférieur en C₂-C₆ et/ou d'un polyol tel que le glycérol, le butylèneglycol, l'isoprèneglycol, le propylèneglycol, le polyéthylèneglycol.

La composition selon l'invention peut comprendre une phase grasse, notamment constituée de corps gras liquides à 25°C, tels que des huiles d'origine animale, végétale, minérale ou synthétique.
Lorsque la composition selon l'invention se présente sous la forme d'une émulsion, ladite phase grasse peut comprendre toute huile cosmétiquement acceptable, dans la mesure où ladite huile permet, en mélange avec la phase aqueuse et les éventuels additifs, l'obtention d'une émulsion stable, c'est-à-dire d'une émulsion qui ne casse pas, qui reste sous forme d'une phase unique pendant au moins 24 heures après stockage à 25°C, sans phénomène de crémage ou de relarguage d'huile.

Les huiles susceptibles d'être employées peuvent éventuellement être volatiles à température ambiante (20-25°C). On entend par huile volatile, tout composé susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.
Ces composés volatils peuvent être choisis en particulier parmi les huiles hydrocarbonées et/ou les huiles siliconées, cycliques ou linéaires, seules ou en mélange. On peut ainsi citer les huiles siliconées volatiles, telles que :
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE, qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane ou un PDMS de faible viscosité (1 cSt). On peut encore citer les alkyltrisiloxanes tels que l'hexylheptaméthyltrisiloxane ou l'octylheptaméthyltrisiloxane.
On peut également citer les huiles volatiles hydrocarbonées, telles que les isoparaffines et notamment l'isododécane.

Parmi les huiles non volatiles, on peut citer :
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment celles de formule : 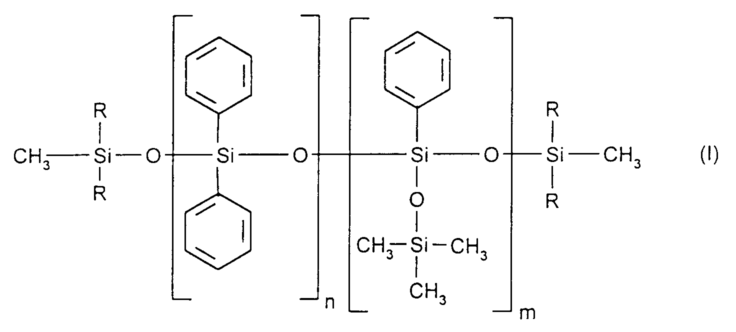 dans laquelle R est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100;
- les huiles d'origine animale, végétale ou minérale, telles que l'huile de paraffine, de vaseline, le perhydrosqualène, l'huile d'abricot, l'huile de germes de blé, d'amande douce, de calophyllum, de sésame, de macadamia, de pépins de raisin, de colza, de coprah, d'arachide, de palme, de ricin, d'avocat, de jojoba, d'olive ou de germes de céréales; des esters d'acides gras et de polyol, en particulier les triglycérides liquides; des alcools; des acétylglycérides; des octanoates, décanoates ou ricinoléates d'alcools ou de polyalcools; des triglycérides d'acides gras; des glycérides, les huiles fluorées et perfluorées;
- leurs mélanges.

Dans un mode de réalisation particulier de l'invention, on peut préparer une émulsion ne comprenant que des corps gras siliconés, tels que
- des huiles cycliques volatiles éventuellement en mélange avec des PDMS et/ou des huiles siliconées phénylées, ou
- des gommes de silicone, notamment phénylées et/ou hydroxylées, en mélange avec des huiles siliconées éventuellement volatiles.
De préférence, la composition comprend au maximum environ 10% en poids d'huile hydrocarbonée non volatile; notamment la composition peut comprendre moins de 8% en poids d'huile hydrocarbonée non volatile, plus préférentiellement moins de 5% en poids, voire pas d'huile hydrocarbonée non volatile du tout.

Lorsque la composition se présente sous forme d'une émulsion huile-dans-eau, la phase grasse de l'émulsion peut être présente à une teneur de 2 % à 40 % en poids par rapport au poids total de l'émulsion, de préférence de 3 % à 30 % en poids et en particulier de 3 % à 20 % en poids.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance, en texture et/ou en transfert. Ces corps gras additionnels peuvent être des cires, des gommes et/ou des corps gras pâteux d'origine animale, végétale, minérale ou synthétique, ainsi que leurs mélanges.
On peut notamment citer :
- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées; leurs mélanges.

La composition selon l'invention peut en outre comprendre un ou plusieurs solvants organiques cosmétiquement acceptables (tolérance, toxicologie et toucher acceptables). Ces solvants organiques peuvent représenter de 0 % à 98 % du poids total de la composition. Ils peuvent être choisis dans le groupe constitué par les solvants organiques hydrophiles, les solvants organiques lipophiles, les solvants amphiphiles ou leurs mélanges.
Parmi les solvants organiques hydrophiles, on peut citer par exemple des monoalcools inférieurs linéaires ou ramifiés ayant 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono-ou di-alkyle d'isosorbide dont les groupements alkyle ont de 1 à 5 atomes de carbone ; les éthers de glycol comme le diéthylène glycol mono-méthyl ou mono-éthyl éther et les éthers de propylène glycol comme le dipropylène glycol méthyl éther. Comme solvants organiques amphiphiles, on peut citer des polyols tels des dérivés de polypropylène glycol (PPG) tels que les esters de polypropylène glycol et d'acide gras, de PPG et d'alcool gras comme le PPG-23 oléyl éther et le PPG-36 oléate. Comme solvants organiques lipophiles, on peut citer par exemple les esters gras tels que l'adipate de diisopropyle, l'adipate de dioctyle, les benzoates d'alkyle.

Lorsque la composition selon l'invention se présente sous la forme d'une émulsion, elle peut éventuellement comprendre en outre un tensioactif, bien que cela ne soit pas nécessaire pour l'obtention d'une émulsion stable et fine. Le tensioactif permet toutefois d'affiner l'émulsion obtenue. Comme tensioactif H/E, on peut citer notamment (CTFA) : le cétéaryl-glucoside, le PEG-40 stéarate, le sorbitan tristéarate, le sorbitan stéarate, le polysorbate 60, le mélange sorbitan stéarate/sucrose cocoate, le mélange de glycéryl stéarate/PEG-100 stéarate, le PEG-400, le stéarate de glycéryle, le mélange de PEG-6/PEG-32/glycol stéarate. Comme tensioactif E/H, on peut citer notamment le mélange polyglycéryl-4 isostéarate/ cétyldiméthicone copolyol/ hexyl laurate et le mélange Mineral oil/petrolatum/ozokérite/glycéryl oléate/alcool de lanoline.
Elle peut également comprendre 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle ou le polyglycéryl 10-décaoléate.

En outre, l'émulsion selon l'invention peut comprendre un ou plusieurs agents épaississants dans des concentrations préférentielles de 0 à 6 % en poids, par rapport au poids total de l'émulsion. L'agent épaississant peut être choisi parmi :
- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose,
- les polymères synthétiques comme les acides polyacryliques tels que les polymères poly(méth)acrylates de glycéryl tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST; les copolymères acrylate/octylacrylamide tels que le Dermacryl de National Starch; les polymères à base de polyacrylamide tels que le SEPIGEL 305 de SEPPIC, les polymères réticulés d'acrylamide et de chlorure de méthacryloyloxyéthyltriméthylammonium tels que le SALCARE SC 92 de ALLIED COLLOIDS,
- le silicate de magnésium et d'aluminium.
- les épaississants inorganiques tels que les smectites, les hectorites modifiées ou non (BENTONE, LAPONITE par exemple),
- leurs mélanges.
D'une manière préférée, on utilise comme gélifiant le polymère poly(acide 2-acrylamido 2-méthylpropane sulfonique) réticulé et neutralisé à au moins 90%, tel que défini ci-dessus.
Dans un autre mode de réalisation préféré, on utilise comme polymère épaississant un homo ou copolymère d'acide acrylique, éventuellement réticulé, ou un de ses sels, tel que ceux commercialisés sous le nom 'CARBOPOL' par la société GOODRICH. Un tel polymère épaississant permet en effet notamment d'obtenir un produit physiquement et cosmétiquement agréable, notamment lisse et d'application satisfaisante, avec une viscosité pouvant aller du lait fluide à la crème.

La composition selon l'invention peut comprendre en outre une phase particulaire qui peut comprendre des pigments et/ou des nacres et/ou des charges habituellement utilisés dans les compositions cosmétiques. Par pigments, il faut comprendre des particules blanches ou colorées, minérales ou organiques, insolubles dans le milieu, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.
Les pigments peuvent être présents à raison de 0-20 % en poids, par rapport au poids total de la composition, et de préférence à raison de 2-15 %. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes, le bleu ferrique. Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques.
Lorsque la composition se présente sous forme d'une émulsion huile-dans-eau, les pigments ont de préférence une surface hydrophobe ou peuvent être traités de manière à rendre leur surface hydrophobe; ce traitement peut être effectué selon les méthodes connues de l'homme du métier; les pigments peuvent notamment être enrobés par des composés siliconés tels que des PDMS et/ou par des polymères, notamment des polyéthylènes. On peut ainsi citer les pigments SA de Maprecos ou les pigments PI de Myoshi. En effet, on a constaté que lorsque les pigments n'étaient pas enrobés, le produit obtenu présentait un aspect non lisse et/ou cassant.
Lorsque la composition se présente sous forme d'une solution ou d'un gel aqueux, elle peut comprendre des colorants hydrosolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

Les nacres peuvent être présentes dans la composition à raison de 0-20% en poids, de préférence à un taux élevé de l'ordre de 2-15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présentes dans la composition à raison de 0-20 % en poids, par rapport au poids total de la composition, de préférence 2-10%, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphéres telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique, tel que des antioxydants, des parfums, des huiles essentielles, des conservateurs, des actifs cosmétiques ou pharmaceutiques lipophiles ou hydrophiles, des hydratants, des vitamines, des acides gras essentiels, des sphingolipides, des composés auto-bronzants tels que la DHA, des filtres solaires. Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.
Ces additifs peuvent être présents dans la composition à raison de 0-10% en poids. Selon leur nature, ils sont présents dans la phase aqueuse ou dans la phase grasse de la composition.

Les compositions selon l'invention peuvent se présenter sous toutes les formes appropriées pour une application topique, notamment sous forme de sérum, de lotion, de crème, de lait, de gel aqueux, d'émulsions obtenues par dispersion d'une phase grasse dans une phase aqueuse (H/E) ou inversement (E/H), de consistance liquide ou semi-liquide, voire pâteuse ou solide.
Les émulsions selon l'invention peuvent constituer tout ou partie d'une composition cosmétique, pharmaceutique ou hygiénique.

Les compositions selon l'invention trouvent une application notamment dans le domaine du maquillage de la peau, des semi-muqueuses, des muqueuses et/ou des phanères, et se présentent alors par exemple sous la forme d'un fond de teint, d'un fard à joues ou à paupières, d'un rouge à lèvres, d'un mascara, d'un eye-liner ou d'un vernis à ongles.
Elles peuvent également être utilisées comme base de soin pour les lèvres, ou comme produit de soin de la peau, des muqueuses, des semi-muqueuses et/ou des phanères, tel qu'un gel, une crème, un baume ou une lotion, ou comme produit hygiénique ou pharmaceutique ou encore comme produit solaire ou autobronzant.
Elles trouvent également une application dans le domaine capillaire, notamment en tant que gels ou crèmes de soin des phanères telles que cheveux, cils et sourcils, ou encore en tant que gel aqueux notamment de coiffage.

L'invention est illustrée plus en détails dans les exemples suivants.

### Exemples 1 à 15 : Etude des propriétés de persistance de différentes émulsions

On a réalisé différentes émulsions (fond de teint) conformes à l'invention, chaque émulsion ayant une composition différente.

Les émulsions comprennent les composés suivants :
. une phase grasse
. le copolymère selon l'invention (PEMULEN TR2 de GOODRICH) et éventuellement :
. un poly(acide 2-acrylamido 2-méthylpropane sulfonique) réticulé et neutralisé par l'ammoniaque, de viscosité à 25°C de 16000 cps en solution aqueuse à 2%
. un copolymère d'acide acrylique, éventuellement réticulé (CARBOPOL 980 de GOODRICH)
. de l'alcool polyvinylique
. des tensioactifs (monooléate de sorbitane 200E; décaoléate de polyglycéryle)
. des charges.
. des pigments enrobés PDMS ou polyéthylène
. des colorants hydrosolubles
. de l'eau qsp 100 g

On a préparé les compositions, de manière usuelle, en mélangeant les ingrédients de la phase grasse et les pigments préalablement dispersés ; on a préparé la phase aqueuse en mélangeant l'eau et les copolymères à 80°C; on a mélangé les deux phases à température ambiante sous agitation à l'aide d'une turbine puis on a laissé refroidir.

### Exemple 1

| | |
|---|---|
| . gomme de silicone hydroxylée dans PDMS (Q2-1403 de Dow Corning) | 16 g |
| . PEMULEN TR2 de GOODRICH | 0,1 g |
| . poly(acide 2-acrylamido 2-méthylpropane sulfonique) | 0,6 g |
| . pigments enrobés PDMS | 10 g |
| . eau | qsp 100 g |

### Exemple 2

| | |
|---|---|
| . gomme de silicone hydroxylée dans PDMS (Q2-1403 de Dow Corning) | 16 g |
| . PEMULEN TR2 de GOODRICH | 0,1 g |
| . poly(acide 2-acrylamido 2-méthylpropane sulfonique) | 0,6 g |
| . alcool polyvinylique | 0,5 g |
| . pigments enrobés PDMS | 10 g |
| . eau | qsp 100 g |

### Exemple 3

| | |
|---|---|
| . gomme de silicone hydroxylée dans PDMS (Q2-1403 de Dow Corning) | 16 g |
| . PEMULEN TR2 de GOODRICH | 0,1 g |
| . poly(acide 2-acrylamido 2-méthylpropane sulfonique) | 0,6 g |
| . alcool polyvinylique | 0,5 g |
| . tensioactif | 1,1 g |
| . pigments enrobés PDMS | 12 g |
| . eau | qsp 100 g |

### Exemple 4

| | |
|---|---|
| . gomme de silicone hydroxylée dans PDMS (Q2-1403 de Dow Corning) | 11 g |
| . cyclopentadiméthylsiloxane (silicone cycloD5, volatile) | 5 g |
| . PEMULEN TR2 de GOODRICH | 0,1 g |
| . poly(acide 2-acrylamido 2-méthylpropane sulfonique) | 0,6 g |
| . alcool polyvinylique | 0,5 g |
| . tensioactif | 1,1 g |
| . pigments enrobés PDMS | 12 g |
| . eau | qsp 100 g |

### Exemple 5

| | |
|---|---|
| . gomme de silicone hydroxylée dans PDMS (Q2-1403 de Dow Corning) | 8 g |
| . huile d'abricot | 8 g |
| . PEMULEN TR2 de GOODRICH | 0,1 g |
| . CARBOPOL 980 de GOODRICH | 0,6 g |
| . tensioactif | 0,1 g |
| . pigments enrobés PDMS | 12 g |
| . eau | qsp 100 g |

### Exemple 6

| | |
|---|---|
| . gomme de silicone hydroxylée dans PDMS (Q2-1403 de Dow Corning) | 13 g |
| . cyclopentadiméthylsiloxane | 3 g |
| . PEMULEN TR2 de GOODRICH | 0,1 g |
| . poly(acide 2-acrylamido 2-méthylpropane sulfonique) | 0,5 g |
| . alcool polyvinylique | 0,5 g |
| . tensioactif | 1,1 g |
| . pigments enrobés PDMS | 14 g |
| . microsphères creuses (Expancel 551 DE20) | 0,5 g |
| . eau | qsp 100 g |

### Exemple 7

| | |
|---|---|
| . gomme de silicone hydroxylée dans PDMS (Q2-1403 de Dow Corning) | 13 g |
| . cyclopentadiméthylsiloxane | 3 g |
| . PEMULEN TR2 de GOODRICH | 0,1 g |
| . poly(acide 2-acrylamido 2-méthylpropane sulfonique) | 0,5 g |
| . alcool polyvinylique | 0,6 g |
| . tensioactif | 1,1 g |
| . pigments enrobés PDMS | 14 g |
| . charge (Nylon) | 0,5 g |
| . eau | qsp 100 g |

### Exemple 8

| | |
|---|---|
| . gomme de silicone hydroxylée dans PDMS (Q2-1403 de Dow Corning) | 16 g |
| . PEMULEN TR2 de GOODRICH | 0,6 g |
| . tensioactif | 1,1 g |
| . pigments enrobés PDMS | 12 g |
| . eau | qsp 100 g |

### Exemple 9

| | |
|---|---|
| . gomme de silicone hydroxylée dans PDMS (Q2-1403 de Dow Corning) | 16 g |
| . PEMULEN TR2 de GOODRICH | 0,1 g |
| . CARBOPOL 980 de GOODRICH | 0,6 g |
| . tensioactif | 0,1 g |
| . pigments enrobés PDMS | 10 g |
| . eau | qsp 100 g |

### Exemple 10

| | |
|---|---|
| . gomme de silicone hydroxylée dans PDMS (Q2-1403 de Dow Corning) | 16 g |
| . PEMULEN TR2 de GOODRICH | 0,1 g |
| . CARBOPOL 980 de GOODRICH | 0,6 g |
| . pigments enrobés polyéthylène | 4 g |
| . eau | qsp 100 g |

### Exemple 11

| | |
|---|---|
| . gomme de silicone hydroxylée dans PDMS (Q2-1403 de Dow Corning) | 16 g |
| . PEMULEN TR2 de GOODRICH | 0,1 g |
| . CARBOPOL 980 de GOODRICH | 0,6 g |
| . colorants hydrosolubles | 0,274 g |
| . eau | qsp 100 g |

### Exemple 12

| | |
|---|---|
| . PDMS (10 cSt) | 10 g |
| . huile d'abricot | 10 g |
| . PEMULEN TR2 de GOODRICH | 0,1 g |
| . poly(acide 2-acrylamido 2-méthylpropane sulfonique) | 0,6 g |
| . alcool polyvinylique | 0,6 g |
| . tensioactif | 1,1 g |
| . pigments enrobés PDMS | 14 g |
| . eau | qsp 100 g |

### Exemple 13

| | |
|---|---|
| . PDMS ( 10 cSt) | 15 g |
| . huile d'abricot | 10 g |
| . PEMULEN TR2 de GOODRICH | 0,1 g |
| . poly(acide 2-acrylamido 2-méthylpropane sulfonique) | 0,6 g |
| . alcool polyvinylique | 0,6 g |
| . tensioactif | 1,1 g |
| . pigments enrobés PDMS | 14 g |
| . eau | qsp 100 g |

### Exemple 14

| | |
|---|---|
| . PDMS (10 cSt) | 15 g |
| . PEMULEN TR2 de GOODRICH | 0,1 g |
| . poly(acide 2-acrylamido 2-méthylpropane sulfonique) | 0,6 g |
| . alcool polyvinylique | 0,6 g |
| . tensioactif | 1,1 g |
| . pigments enrobés PDMS | 14 g |
| . eau | qsp 100 g |

### Exemple 15

| | |
|---|---|
| . PDMS (10 cSt) | 6 g |
| . huile d'abricot | 10 g |
| . PEMULEN TR2 de GOODRICH | 0,1 g |
| . poly(acide 2-acrylamido 2-méthylpropane sulfonique) | 0,6 g |
| . alcool polyvinylique | 0,6 g |
| . tensioactif | 1,1 g |
| . pigments enrobés PDMS | 14 g |
| . eau | qsp 100 g |

On obtient ainsi des fonds de teint qui s'étalent facilement sur la peau sans sensation de gras et ne transfèrent pas au contact d'un tissu.
Ces fonds de teint confèrent une certaine sensation de fraîcheur lors de leur application sur la peau.

On a ensuite déterminé les propriétés de persistance de ces émulsions.
Pour cela, on a appliqué 0,05 g de chaque émulsion sur une surface de 50 cm² sur l'avant-bras puis on a laissé sécher la composition appliquée pendant 5 minutes. On a ensuite appliqué une bande de tissu en polyester sur la partie de l'avant-bras traité. A l'aide d'un appareil, on a animé la bande d'un mouvement de translation vertical, au contact de l'avant-bras traité. Le tissu a été maintenu tendu à l'aide d'un contrepoids créant ainsi un frottement du tissu pendant la translation. On a réalisé 10 mouvements aller et retour de frottement.

On a ensuite noté les traces colorées qui se sont éventuellement déposées sur le tissu selon la notation suivante :
tissu très taché : note = 0
tissu sans trace : note = 10
On considère qu'un fond de teint transfère peu lorsque la notation est égale ou supérieure à 8,5.
Les résultats obtenus sont reportés dans le tableau ci-après.

| Exemple | Note |
|---|---|
| Exemple 1 | 9 |
| Exemple 2 | 9,5 |
| Exemple 3 | 9 |
| Exemple 4 | 9,5 |
| Exemple 5 | 10 |
| Exemple 6 | 9 |
| Exemple 7 | 9,5 |
| Exemple 8 | 9,5 |
| Exemple 9 | 10 |
| Exemple 10 | 8,5 |
| Exemple 11 | 9,5 |
| Exemple 12 | 8,5 |
| Exemple 13 | 9 |
| Exemple 14 | 8,5 |
| Exemple 15 | 8,5 |

II en ressort que les émulsions selon l'invention présentent de bonnes propriétés de persistance et ne transfèrent pas sur le tissu.
Ceci est également le cas lorsque des huiles d'origine végétale (huile d'abricot) sont présentes dans la composition, à une teneur pouvant aller jusqu'à 10% en poids.

Les exemples 1 et 2, directement comparables, montrent également que l'association PEMULEN TR2-Alcool polyvinylique est particulièrement performante pour limiter le transfert d'une composition selon l'invention.

### Exemple 16 : exemple comparatif

Pour comparaison, on a préparé la composition suivante :

| | |
|---|---|
| . gomme de silicone hydroxylée dans PDMS (Q2-1403 de Dow Corning) | 16 g |
| . CARBOPOL 980 de GOODRICH | 0,7 g |
| . pigments enrobés PDMS | 12 g |
| . eau | qsp 100 g |

On a constaté qu'il n'était pas possible de disperser les corps gras, le Carbopol n'étant pas en quantité suffisante.

### Exemples 17 et 18 :

Ces exemples sont destinés à montrer que la présence d'une quantité importante d'huile hydrocarbonée (végétale dans le cas présent) est défavorable au non-transfert.

Ces exemples sont à comparer aux exemples 3 et 4 précédents.

### Exemple 17

| | |
|---|---|
| . gomme de silicone hydroxylée dans PDMS (Q2-1403 de Dow Corning) | 13 g |
| . huile d'abricot | 12 g |
| . PEMULEN TR2 de GOODRICH | 0,1 g |
| . poly(acide 2-acrylamido 2-méthylpropane sulfonique) | 0,6 g |
| . alcool polyvinylique | 0,6 g |
| . tensioactif | 1,1 g |
| . pigments enrobés PDMS | 14 g |
| . eau | qsp 100 g |

### Exemple 18

| | |
|---|---|
| . gomme de silicone hydroxylée dans PDMS (Q2-1403 de Dow Corning) | 13 g |
| . néopentanoate d'isostéaryle | 12 g |
| . PEMULEN TR2 de GOODRICH | 0,1 g |
| . poly(acide 2-acrylamido 2-méthylpropane sulfonique) | 0,6 g |
| . alcool polyvinylique | 0,6 g |
| . tensioactif | 1,1 g |
| . pigments enrobés PDMS | 14 g |
| . eau | qsp 100 g |

Pour ces exemples 17 et 18, on a déterminé les propriétés de persistance des émulsions selon le test décrit ci-dessus. On obtient les résultats suivants:

| Exemple | Note |
|---|---|
| Exemple 17 | 6 |
| Exemple 18 | 6,5 |

### Exemple 19 : comparatif

Cet exemple est destiné à montrer l'amélioration apportée, au niveau de la limitation du transfert d'une composition, par l'utilisation de PEMULEN TR2 : on a ainsi réalisé un fond de teint A conforme à l'invention et comprenant du PEMULEN TR2 et un fond de teint B de même composition que A mais dans lequel on a remplacé le PEMULEN TR2 par un système émulsionnant classique (acide stéarique/stéarate)-triéthanolamine. Les compositions sont données ci-après :

### Fond de teint A :

| | |
|---|---|
| . PDMS (10 cSt) | 10 g |
| . huile d'abricot | 10 g |
| . PEMULEN TR2 de GOODRICH | 0,1 g |
| . poly(acide 2-acrylamido 2-méthylpropane sulfonique) | 0,6 g |
| . alcool polyvinylique | 0,6 g |
| . tensioactif | 1,1 g |
| . pigments enrobés PDMS | 14 g |
| . eau | qsp 100 g |

### Fond de teint B :

| | |
|---|---|
| . PDMS (10 cSt) | 10 g |
| . huile d'abricot | 10 g |
| . acide stéarique/stéarate | 4,4 g |
| . triéthanolamine | 1,1 g |
| . poly(acide 2-acrylamido 2-méthylpropane sulfonique) | 0,6 g |
| . alcool polyvinylique | 0,6 g |
| . tensioactif | 1,1 g |
| . pigments enrobés PDMS | 14 g |
| . eau | qsp 100 g |

Ces fonds de teint ont été préparés de manière classique en mélangeant les ingrédients de la phase grasse et les pigments dispersés. Puis on a préparé la phase aqueuse et on a mélangé les deux phases à 65°C sous agitation à l'aide d'une turbine. On a ensuite laissé refroidir le mélange.

On a ensuite déterminé les propriétés de persistance de ces deux fonds de teint selon le même protocole que dans les exemples 1 à 15. Les résultats obtenus sont reportés dans le tableau ci-dessous :

| Fond de teint | Note |
|---|---|
| A (invention) | 9 |
| B (comparatif) | 6 |

## Revendications

1. Utilisation d'un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique dans une composition cosmétique, dermatologique, hygiénique et/ou pharmaceutique, afin de limiter, diminuer et/ou de supprimer le transfert et/ou la migration de ladite composition.

2. Utilisation d'un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique, dans une composition cosmétique, dermatologique, hygiénique et/ou pharmaceutique, afin d'améliorer la tenue de ladite composition.

3. Utilisation d'un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique dans une composition cosmétique, dermatologique, hygiénique et/ou pharmaceutique, comme agent permettant de limiter, diminuer et/ou de supprimer le transfert et/ou la migration de ladite composition, et/ou comme agent permettant d'améliorer la tenue de ladite composition.

4. Utilisation d'un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique dans une composition cosmétique, dermatologique, hygiénique et/ou pharmaceutique, pour former un film de ladite composition, ce film ne transférant et/ou ne migrant pas et/ou ayant une tenue améliorée.

5. Utilisation d'un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique dans une composition sans transfert.

6. Utilisation selon l'une des revendications précédentes, dans laquelle le copolymère est présent dans la composition à une concentration de 0,01 à 3% en poids par rapport au poids total de la composition, de préférence 0,02 à 0,6% en poids, et plus préférentiellement de 0,05 à 0,2% en poids.

7. Utilisation selon l'une des revendications précédentes, dans laquelle la composition comprend en outre
. un polymère poly(acide 2-acrylamido 2-méthylpropane sulfonique) réticulé et neutralisé à au moins 90% et/ou
. un homo ou copolymère d'acide acrylique, éventuellement réticulé, ou un de ses sels.

8. Utilisation selon l'une quelconque des revendications précédentes dans laquelle la composition comprend en outre de l'alcool polyvinylique.

9. Utilisation selon l'une des revendications précédentes, dans laquelle la composition se présente sous la forme d'une émulsion huile-dans-eau, d'une émulsion eau-dans-huile, d'une émulsion multiple, d'un gel aqueux, ou d'une solution aqueuse, hydroalcoolique ou multiphasée, notamment eau/poudre et eau/huile/poudres.

10. Utilisation selon l'une des revendications précédentes, dans laquelle la composition comprend une phase grasse qui comprend au maximum environ 10% en poids d'huile hydrocarbonée non volatile; de préférence moins de 8% en poids, plus préférentiellement moins de 5% en poids, voire pas d'huile hydrocarbonée non volatile du tout.

11. Utilisation selon l'une des revendications 9 à 10, dans laquelle la composition comprend une phase grasse qui ne comprend que des corps gras siliconés, tels que
- des huiles cycliques volatiles éventuellement en mélange avec des PDMS et/ou des huiles siliconées phénylées, ou
- des gommes de silicone, notamment phénylées et/ou hydroxylées, en mélange avec des huiles siliconées éventuellement volatiles.

12. Utilisation selon l'une des revendications précédentes, dans laquelle la composition se présente sous la forme d'une composition de maquillage de la peau, des semi-muqueuses, des muqueuses et/ou des phanères, telle qu'un fond de teint, un fard à joues ou à paupières, un rouge à lèvres, un mascara, un eye-liner, un vernis à ongles; une base de soin pour les lèvres; un produit de soin de la peau, des muqueuses, des semi-muqueuses et/ou des phanères; un produit hygiénique ou pharmaceutique; un produit solaire ou autobronzant; un produit capillaire.

13. Composition cosmétique, dermatologique, hygiénique et/ou pharmaceutique, se présentant sous la forme de, et/ou comprenant, une émulsion huile-dans-eau, une émulsion eau-dans-huile, une émulsion multiple ou une solution multiphasée, comprenant en outre au moins un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique, et des pigments ayant une surface hydrophobe.

14. Composition cosmétique, dermatologique, hygiénique et/ou pharmaceutique, se présentant sous la forme de, et/ou comprenant, un gel aqueux, une solution aqueuse, hydroalcoolique ou multiphasée, comprenant en outre au moins un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique et des colorants hydrosolubles.

15. Composition sans transfert pour le maquillage et/ou le soin de la peau, des semi-muqueuses, des muqueuses et/ou des phanères, comprenant au moins un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique.

16. Composition selon l'une des revendications 13 à 15, dans laquelle la quantité de monomère acide carboxylique ou de son anhydride dans le copolymère est comprise entre 80 et 98% en poids et plus particulièrement entre 90 et 98% en poids; et la quantité de monomère ester acrylique est comprise entre 2 et 20% en poids et plus particulièrement entre 2 et 10% en poids.

17. Composition selon l'une des revendications 13 à 16, dans laquelle le copolymère est présent à une concentration de 0,01 à 3% en poids par rapport au poids total de la composition, de préférence 0,02 à 0,6% en poids, et plus préférentiellement de 0,05 à 0,2% en poids.

18. Composition selon l'une des revendications 13 à 17, comprenant en outre un polymère poly(acide 2-acrylamido 2-méthylpropane sulfonique) réticulé et neutralisé à au moins 90%.

19. Composition selon la revendication 18, dans laquelle le polymère poly(acide 2-acrylamido 2-méthylpropane sulfonique) est présent à une concentration de 0 à 20% en poids par rapport au poids total de la composition, de préférence 0,1 à 5% en poids, et plus préférentiellement 0,4 à 2% en poids.

20. Composition selon l'une des revendications 13 à 19, comprenant en outre un homo ou copolymère d'acide acrylique, éventuellement réticulé ou un de ses sels.

21. Composition selon l'une quelconque des revendications 13 à 20, comprenant en outre de l'alcool polyvinylique.

22. Composition selon l'une des revendications 13 à 21, se présentant sous la forme d'une émulsion huile-dans-eau, d'une émulsion eau-dans-huile, d'une émulsion multiple, d'un gel aqueux, ou d'une solution aqueuse, hydroalcoolique ou multiphasée, notamment eau/poudre et eau/huile/poudres.

23. Composition selon l'une des revendications 13 à 22, dans laquelle la composition comprend une phase grasse qui comprend au maximum environ 10% en poids d'huile hydrocarbonée non volatile; de préférence moins de 8% en poids d'huile hydrocarbonée non volatile, plus préférentiellement moins de 5% en poids, voire pas d'huile hydrocarbonée non volatile du tout.

24. Composition selon l'une des revendications 13 à 23, dans laquelle la composition comprend une phase grasse qui ne comprend que des corps gras siliconés, tels que
- des huiles cycliques volatiles éventuellement en mélange avec des PDMS et/ou des huiles siliconées phénylées, ou
- des gommes de silicone, notamment phénylées et/ou hydroxylées, en mélange avec des huiles siliconées éventuellement volatiles.

25. Composition selon l'une des revendications 13 et 15 à 24, dans laquelle les pigments sont présents à raison de 0-20 % en poids, par rapport au poids total de la composition, de préférence à raison de 2-15 % en poids.

26. Composition selon la revendication 25, dans laquelle les pigments sont choisis parmi les pigments et les nanopigments de dioxyde de titane, de zirconium ou de cérium, d'oxydes de zinc, de fer ou de chrome, le bleu ferrique, le noir de carbone, et les sels de calcium, de baryum, d'aluminium ou de zirconium, de colorants acides tels que les colorants halogéno-acides, azoïques ou anthraquinoniques.

27. Composition selon la revendication 25, dans laquelle les pigments sont enrobés par des composés siliconés tels que des PDMS et/ou par des polymères, notamment des polyéthylènes.

28. Composition selon l'une des revendications 14 à 27, dans laquelle le colorant hydrosoluble est choisi parmi le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

29. Composition selon l'une des revendications 13 à 28, se présentant sous la forme d'une composition de maquillage de la peau, des semi-muqueuses, des muqueuses et/ou des phanères, telle qu'un fond de teint, un fard à joues ou à paupières, un rouge à lèvres, un mascara, un eye-liner, un vernis à ongles; une base de soin pour les lèvres; un produit de soin de la peau, des muqueuses, des semi-muqueuses et/ou des phanères; un produit hygiénique ou pharmaceutique; un produit solaire ou autobronzant; un produit capillaire.

30. Fond de teint sans transfert comprenant au moins un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique.

31. Procédé pour limiter, diminuer et/ou empêcher le transfert d'une composition cosmétique, dermatologique, hygiénique et/ou pharmaceutique, notamment une composition de maquillage et/ou de soin de la peau, des muqueuses, des semi-muqueuses et/ou des phanères, consistant à introduire dans ladite composition au moins un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique.

32. Procédé de maquillage de la peau, des semi-muqueuses, des muqueuses et/ou des phanères consistant à appliquer sur sur celles-ci une composition cosmétique telle que définie à l'une quelconque des revendications 13 à 29 ou un fond de teint tel que défini à la revendication 30.

## Claims

1. Use of an optionally crosslinked copolymer consisting of a major fraction of monoolefinically unsaturated C₃-C₆ carboxylic acid monomer or its anhydride and of a minor fraction of fatty-chain monomeric ester of acrylic acid in a cosmetic, dermatological, hygiene and/or pharmaceutical composition, in order to limit, decrease and/or remove altogether the transfer and/or migration of the said composition.

2. Use of an optionally crosslinked copolymer consisting of a major fraction of monoolefinically unsaturated C₃-C₆ carboxylic acid monomer or its anhydride and of a minor fraction of fatty-chain monomeric ester of acrylic acid in a cosmetic, dermatological, hygiene and/or pharmaceutical composition, in order to improve the staying power of the said composition.

3. Use of an optionally crosslinked copolymer consisting of a major fraction of monoolefinically unsaturated C₃-C₆ carboxylic acid monomer or its anhydride and of a minor fraction of fatty-chain monomeric ester of acrylic acid in a cosmetic, dermatological, hygiene and/or pharmaceutical composition, as an agent for limiting, decreasing and/or removing altogether the transfer and/or migration of the said composition, and/or as an agent for improving the staying power of the said composition.

4. Use of an optionally crosslinked copolymer consisting of a major fraction of monoolefinically unsaturated C₃-C₆ carboxylic acid monomer or its anhydride and of a minor fraction of fatty-chain monomeric ester of acrylic acid in a cosmetic, dermatological, hygiene and/or pharmaceutical composition, to form a film of the said composition, this film not transferring and/or migrating and being of improved staying power.

5. Use of an optionally crosslinked copolymer consisting of a major fraction of monoolefinically unsaturated C₃-C₆ carboxylic acid monomer or its anhydride and of a minor fraction of fatty-chain monomeric ester of acrylic acid in a transfer-resistant composition.

6. Use according to one of the preceding claims, in which the copolymer is present in the composition at a concentration of from 0.01 to 3% by weight relative to the total weight of the composition, preferably 0.02 to 0.6% by weight and more preferably 0.05 to 0.2% by weight.

7. Use according to one of the preceding claims, in which the composition also comprises:
• a crosslinked and at least 90% neutralized poly(2-acrylamido-2-methylpropanesulphonic acid) polymer and/or
• an optionally crosslinked acrylic acid homo- or copolymer, or one of the salts thereof.

8. Use according to any one of the preceding claims, in which the composition also comprises polyvinyl alcohol.

9. Use according to one of the preceding claims, in which the composition is in the form of an oil-in-water emulsion, a water-in-oil emulsion, a multiple emulsion, an aqueous gel or an aqueous, aqueous-alcoholic or multi-phase solution, in particular water/powder and water/oil/powders.

10. Use according to one of the preceding claims, in which the composition comprises a fatty phase which comprises a maximum of about 10% by weight of non-volatile hydrocarbon oil, preferably less than 8% by weight, more preferably less than 5% by weight, or even no non-volatile hydrocarbon oil at all.

11. Use according to either of Claims 9 and 10, in which the composition comprises a fatty phase which comprises only silicone fatty substances, such as
- volatile cyclic oils optionally mixed with PDMSs and/or phenylsilicone oils, or
- silicone gums, in particular phenylated and/or hydroxylated silicone gums, mixed with optionally volatile silicone oils.

12. Use according to one of the preceding claims, in which the composition is in the form of a composition for making up the skin, semi-mucous membranes, mucous membranes and/or superficial body growths, such as a foundation, a blusher, an eye-shadow, a lipstick, a mascara, an eye-liner or a nail varnish; a care base for the lips; a care product for the skin, mucous membranes, semi-mucous membranes and/or superficial body growths; a hygiene or pharmaceutical product; an antisun or self-tanning product; a haircare product.

13. Cosmetic, dermatological, hygiene and/or pharmaceutical composition in the form of, and/or comprising, an oil-in-water emulsion, a water-in-oil emulsion, a multiple emulsion or a multi-phase solution, also comprising at least one optionally crosslinked copolymer consisting of a major fraction of monoolefinically unsaturated C₃-C₆ carboxylic acid monomer or its anhydride and of a minor fraction of fatty-chain monomeric ester of acrylic acid, and pigments having a hydrophobic surface.

14. Cosmetic, dermatological, hygiene and/or pharmaceutical composition in the form of, and/or comprising, an aqueous gel or an aqueous, aqueous-alcoholic or multi-phase solution, also comprising at least one optionally crosslinked copolymer consisting of a major fraction of monoolefinically unsaturated C₃-C₆ carboxylic acid monomer or its anhydride and of a minor fraction of fatty-chain monomeric ester of acrylic acid, and water-soluble dyes.

15. Transfer-resistant composition to make up and/or care for the skin, semi-mucous membranes, mucous membranes and/or superficial body growths, comprising at least one optionally crosslinked copolymer consisting of a major fraction of monoolefinically unsaturated C₃-C₆ carboxylic acid monomer or its anhydride and of a minor fraction of fatty-chain monomeric ester of acrylic acid.

16. Composition according to one of Claims 13 to 15, in which the amount of carboxylic acid monomer or its anhydride in the copolymer is between 80 and 98% by weight and more particularly between 90 and 98% by weight; and the amount of acrylic ester monomer is between 2 and 20% by weight and more particularly between 2 and 10% by weight.

17. Composition according to one of Claims 13 to 16, in which the copolymer is present at a concentration of from 0.01 to 3% by weight relative to the total weight of the composition, preferably 0.02 to 0.6% by weight and more preferably 0.05 to 0.2% by weight.

18. Composition according to one of Claims 13 to 17, also comprising a crosslinked and at least 90% neutralized poly(2-acrylamido-2-methylpropanesulphonic acid) polymer.

19. Composition according to Claim 18, in which the poly(2-acrylamido-2-methylpropanesulphonic acid) polymer is present at a concentration of from 0 to 20% by weight relative to the total weight of the composition, preferably 0.1 to 5% by weight and more preferably 0.4 to 2% by weight.

20. Composition according to one of Claims 13 to 19, also comprising an optionally crosslinked acrylic acid homo- or copolymer, or one of its salts.

21. Composition according to any one of Claims 13 to 20, also comprising polyvinyl alcohol.

22. Composition according to one of Claims 13 to 21, in the form of an oil-in-water emulsion, a water-in-oil emulsion, a multiple emulsion, an aqueous gel or an aqueous, aqueous-alcoholic or multi-phase solution, in particular water/powder and water/oil/powders.

23. Composition according to one of Claims 13 to 22, in which the composition comprises a fatty phase which comprises a maximum of about 10% by weight of non-volatile hydrocarbon oil, preferably less than 8% by weight of non-volatile hydrocarbon oil, more preferably less than 5% by weight, or even no non-volatile hydrocarbon oil at all.

24. Composition according to one of Claims 13 to 23, in which the composition comprises a fatty phase which comprises only silicone fatty substances, such as
- volatile cyclic oils optionally mixed with PDMSs and/or phenylsilicone oils, or
- silicone gums, in particular phenylated and/or hydroxylated silicone gums, mixed with optionally volatile silicone oils.

25. Composition according to one of Claims 13 and 15 to 24, in which the pigments are present in a proportion of 0 - 20% by weight relative to the total weight of the composition, preferably in a proportion of 2 - 15% by weight.

26. Composition according to Claim 25, in which the pigments are chosen from pigments and nanopigments of titanium dioxide, zirconium dioxide or cerium dioxide, zinc oxide, iron oxide or chromium oxide, ferric blue, carbon black and the calcium, barium, aluminium or zirconium salts of acidic dyes such as haloacid dyes, azo dyes or anthraquinone dyes.

27. Composition according to Claim 25, in which the pigments are coated with silicone compounds such as PDMSs and/or with polymers, in particular polyethylenes.

28. Composition according to one of Claims 14 to 27, in which the water-soluble dye is chosen from the disodium salt of pumice, the disodium salt of alizarin green, quinoline yellow, the trisodium salt of amaranth, the disodium salt of tartrazine, the monosodium salt of rhodamine, the disodium salt of fuchsin and xanthophyll.

29. Composition according to one of Claims 13 to 28, in the form of a composition for making up the skin, semi-mucous membranes, mucous membranes and/or superficial body growths, such as a foundation, a blusher, an eye-shadow, a lipstick, a mascara, an eye-liner or a nail varnish; a care base for the lips; a care product for the skin, mucous membranes, semi-mucous membranes and/or superficial body growths; a hygiene or pharmaceutical product; an antisun or self-tanning product; a haircare product.

30. Transfer-resistant foundation comprising at least one optionally crosslinked copolymer consisting of a major fraction of monoolefinically unsaturated C₃-C₆ carboxylic acid monomer or its anhydride and of a minor fraction of fatty-chain monomeric ester of acrylic acid.

31. Process for limiting, decreasing and/or preventing the transfer of a cosmetic, dermatological, hygiene and/or pharmaceutical composition, in particular a composition to make up and/or care for the skin, mucous membranes, semi-mucous membranes and/or superficial body growths, which consists in introducing into the said composition at least one optionally crosslinked copolymer consisting of a major fraction of monoolefinically unsaturated C₃-C₆ carboxylic acid monomer or its anhydride and of a minor fraction of fatty-chain monomeric ester of acrylic acid.

32. Make-up process for the skin, semi-mucous membranes, mucous membranes and/or superficial body growths, which consists in applying to the latter a cosmetic composition as defined in any one of Claims 13 to 29 or a foundation as defined in Claim 30.

## Patentansprüche

1. Verwendung eines gegebenenfalls vernetzten Copolymers, das zum überwiegenden Teil aus Monomeren einer einfach olefinisch ungesättigten Carbonsäure mit 3 bis 6 Kohlenstoffatomen oder des Anhydrids dieser Säure und zu einem kleineren Teil aus Monomeren eines Acrylsäureesters mit Fettkette besteht, in einer kosmetischen, dermatologischen, hygienischen und/oder pharmazeutischen Zusammensetzung, um den Transfer und/oder die Migration der Zusammensetzung abzuschwächen, zu vermindern und/oder zu unterbinden.

2. Verwendung eines gegebenenfalls vernetzten Copolymers, das zum überwiegenden Teil aus Monomeren einer einfach olefinisch ungesättigten Carbonsäure mit 3 bis 6 Kohlenstoffatomen oder des Anhydrids dieser Säure und zu einem kleineren Teil aus Monomeren eines Acrylsäureesters mit Fettkette besteht, in einer kosmetischen, dermatologischen, hygienischen und/oder pharmazeutischen Zusammensetzung zur Verbesserung der Haftung der Zusammensetzung.

3. Verwendung eines gegebenenfalls vernetzten Copolymers, das zum überwiegenden Teil aus Monomeren einer einfach olefinisch ungesättigten Carbonsäure mit 3 bis 6 Kohlenstoffatomen oder des Anhydrids dieser Säure und zu einem kleineren Teil aus Monomeren eines Acrylsäureesters mit Fettkette besteht, in einer kosmetischen, dermatologischen, hygienischen und/oder pharmazeutischen Zusammensetzung als Mittel, mit dem der Transfer und/oder die Migration der Zusammensetzung abgeschwächt, vermindert und/oder unterbunden werden kann, und/oder als Mittel, mit dem die Haftung der Zusammensetzung verbessert werden kann.

4. Verwendung eines gegebenenfalls vernetzten Copolymers, das zum überwiegenden Teil aus Monomeren einer einfach olefinisch ungesättigten Carbonsäure mit 3 bis 6 Kohlenstoffatomen oder des Anhydrids dieser Säure und zu einem kleineren Teil aus Monomeren eines Acrylsäureesters mit Fettkette besteht, in einer kosmetischen, dermatologischen, hygienischen und/oder pharmazeutischen Zusammensetzung zur Bildung eines Films aus der Zusammensetzung, wobei dieser Film sich nicht überträgt und/oder keine Migration zeigt und/oder besser haftet.

5. Verwendung eines gegebenenfalls vernetzten Copolymers, das zum überwiegenden Teil aus Monomeren einer einfach olefinisch ungesättigten Carbonsäure mit 3 bis 6 Kohlenstoffatomen oder des Anhydrids dieser Säure und zu einem kleineren Teil aus Monomeren eines Acrylsäureesters mit Fettkette besteht, in einer Zusammensetzung ohne Transfer.

6. Verwendung nach einem der vorhergehenden Ansprüche, worin das Copolymer in der Zusammensetzung in einer Konzentration von 0,01 bis 3 Gew.-%, vorzugsweise von 0,02 bis 0,6 Gew.-% und noch bevorzugter von 0,05 bis 0,2 Gew.-%, bezogen auf das Gesamtgewicht der Zusammensetzung, vorliegt.

7. Verwendung nach einem der vorhergehenden Ansprüche, worin die Zusammensetzung ferner
- ein vernetztes und zu mindestens 90 % neutralisiertes Poly(2-acrylamido-2-methyl-propansulfonsäure)-Polymer und/oder
- ein gegebenenfalls vernetztes Acrylsäurehomopolymer oder Acrylsäurecopolymer oder eines der Salze dieser Polymere enthält.

8. Verwendung nach einem der vorhergehenden Ansprüche, worin die Zusammensetzung ferner Polyvinylalkohol enthält.

9. Verwendung nach einem der vorhergehenden Ansprüche, worin die Zusammensetzung in Form einer Öl-in-Wasser-Emulsion, einer Wasser-in-Öl-Emulsion, einer multiplen Emulsion, eines wässerigen Gels, einer wässerigen oder wässerig-alkoholischen Lösung oder einer Lösung aus mehreren Phasen, insbesondere Wasser/Pulver und Wasser/Öl/Pulver, vorliegt.

10. Verwendung nach einem der vorhergehenden Ansprüche, worin die Zusammensetzung eine Fettphase enthält, die maximal ungefähr 10 Gew.-%, vorzugsweise weniger als 8 Gew.-% und noch bevorzugter weniger als 5 Gew.-% eines nicht flüchtigen Kohlenwasserstofföls oder überhaupt kein nicht flüchtiges Kohlenwasserstofföl enthält.

11. Verwendung nach einem der Ansprüche 9 bis 10, worin die Zusammensetzung eine Fettphase umfaßt, die nur siliconhaltige Fettsubstanzen enthält, wie
- flüchtige cyclische Öle gegebenenfalls im Gemisch mit PDMS und/oder phenylhaltigen Siliconölen, oder
- insbesondere phenylhaltige und/oder hydroxyhaltige Silicongummis im Gemisch mit gegebenenfalls flüchtigen Siliconölen.

12. Verwendung nach einem der vorhergehenden Ansprüche, worin die Zusammensetzung in Form einer Zusammensetzung zum Schminken der Haut, der Semi-Schleimhäute, der Schleimhäute und/oder der Hautanhangsgebilde vorliegt, wie als Make-up, Wangenrouge, Lidschatten, Lippenstift, Mascara, Eyeliner oder Nagellack; als Pflegegrundmasse für die Lippen; als Produkt zur Pflege der Haut, der Schleimhäute, der Semi-Schleimhäute und/oder der Hautanhangsgebilde; als hygienisches oder pharmazeutisches Produkt; als Sonnenschutzprodukt oder selbstbräunendes Produkt oder als Produkt zur Haarbehandlung.

13. Kosmetische, dermatologische, hygienische und/oder pharmazeutische Zusammensetzung, die in Form einer Öl-in-Wasser-Emulsion, einer Wasser-in-Öl-Emulsion, einer multiplen Emulsion oder einer mehrphasigen Lösung vorliegt und/oder die eine Öl-in-Wasser-Emulsion, eine Wasser-in-Öl-Emulsion, eine multiple Emulsion oder eine mehrphasige Lösung enthält, und die ferner mindestens ein gegebenenfalls vernetztes Copolymer, das zum überwiegenden Teil aus Monomeren einer einfach olefinisch ungesättigten Carbonsäure mit 3 bis 6 Kohlenstoffatomen oder des Anhydrids dieser Säure und zu einem kleineren Teil aus Monomeren eines Acrylsäureesters mit Fettkette besteht, und Pigmente enthält, die eine hydrophobe Oberfläche aufweisen.

14. Kosmetische, dermatologische, hygienische und/oder pharmazeutische Zusammensetzung, die in Form eines wässerigen Gels, einer wässerigen oder wässerig-alkoholischen Lösung oder einer Lösung aus mehreren Phasen vorliegt und/oder die ein wässeriges Gel, eine wässerige oder wässerig-alkoholische Lösung oder eine Lösung aus mehreren Phasen enthält, und die ferner mindestens ein gegebenenfalls vernetztes Copolymer, das zum überwiegenden Teil aus Monomeren einer einfach olefinisch ungesättigten Carbonsäure mit 3 bis 6 Kohlenstoffatomen oder des Anhydrids dieser Säure und zu einem kleineren Teil aus Monomeren eines Acrylsäureesters mit Fettkette besteht, und wasserlösliche Färbemittel enthält.

15. Zusammensetzung ohne Transfer zum Schminken und/oder zur Pflege der Haut, der Semi-Schleimhäute, der Schleimhäute und/oder der Hautanhangsgebilde, die mindestens ein gegebenenfalls vernetztes Copolymer enthält, das zum überwiegenden Teil aus Monomeren einer einfach olefinisch ungesättigten Carbonsäure mit 3 bis 6 Kohlenstoffatomen oder des Anhydrids dieser Säure und zu einem kleineren Teil aus Monomeren eines Acrylsäureesters mit Fettkette besteht.

16. Zusammensetzung nach einem der Ansprüche 13 bis 15, worin das Carbonsäuremonomer oder sein Anhydrid in dem Copolymer in einem Mengenanteil im Bereich von 80 bis 98 Gew.-% und insbesondere von 90 bis 98 Gew.-% vorliegt und worin das Acrylsäureestermonomer in einem Mengenanteil von 2 bis 20 Gew.-% und insbesondere von 2 bis 10 Gew.-% vorliegt.

17. Zusammensetzung nach einem der Ansprüche 13 bis 16, worin das Copolymer in einer Konzentration von 0,01 bis 3 Gew.-%, vorzugsweise von 0,02 bis 0,6 Gew.-% und noch bevorzugter von 0,05 bis 0,2 Gew.-%, bezogen auf das Gesamtgewicht der Zusammensetzung, vorliegt.

18. Zusammensetzung nach einem der Ansprüche 13 bis 17, die ferner ein vernetztes und zu mindestens 90 % neutralisiertes Poly(2-acrylamido-2-methyl-propansulfonsäure)-Polymer enthält.

19. Zusammensetzung nach Anspruch 18, worin das Poly(2-acrylamido-2-methyl-propansulfonsäure)-Polymer in einer Konzentration von 0 bis 20 Gew.-%, bezogen auf das Gesamtgewicht der Zusammensetzung, vorzugsweise von 0,1 bis 5 Gew.-% und noch bevorzugter von 0,4 bis 2 Gew.-% vorliegt.

20. Zusammensetzung nach einem der Ansprüche 13 bis 19, die ferner ein gegebenenfalls vernetztes Acrylsäurehomopolymer oder Acrylsäurecopolymer oder eines der Salze dieser Polymere enthält.

21. Zusammensetzung nach einem der Ansprüche 13 bis 20, die ferner Polyvinylalkohol enthält.

22. Zusammensetzung nach einem der Ansprüche 13 bis 21, die in Form einer Öl-in-Wasser-Emulsion, einer Wasser-in-Öl-Emulsion, einer multiplen Emulsion, eines wässerigen Gels, einer wässerigen oder wässerig-alkoholischen Lösung oder einer Lösung aus mehreren Phasen, insbesondere Wasser/Pulver und Wasser/Öl/Pulver, vorliegt.

23. Zusammensetzung nach einem der Ansprüche 13 bis 22, worin die Zusammensetzung eine Fettphase umfaßt, die maximal ungefähr 10 Gew.-% nicht flüchtiges Kohlenwasserstofföl, vorzugsweise weniger als 8 Gew.-% nicht flüchtiges Kohlenwasserstofföl und noch bevorzugter weniger als 5 Gew.-% nicht flüchtiges Kohlenwasserstofföl oder überhaupt kein nicht flüchtiges Kohlenwasserstofföl enthält.

24. Zusammensetzung nach einem der Ansprüche 13 bis 22, worin die Zusammensetzung eine Fettphase umfaßt, die nur siliconhaltige Fettsubstanzen enthält, wie
- flüchtige cyclische Öle gegebenenfalls im Gemisch mit PDMS und/oder phenylhaltigen Siliconölen, oder
- insbesondere phenylhaltige und/oder hydroxyhaltige Silicongummis im Gemisch mit gegebenenfalls flüchtigen Siliconölen.

25. Zusammensetzung nach einem der Ansprüche 13 und 15 bis 24, worin die Pigmente in Mengenanteilen von 0 bis 20 Gew.-%, bezogen auf das Gesamtgewicht der Zusammensetzung, und vorzugsweise in Mengenanteilen von 2 bis 15 Gew.-% vorliegen.

26. Zusammensetzung nach Anspruch 25, worin die Pigmente ausgewählt sind unter den Pigmenten und Nanopigmenten von Titandioxid, Zirconiumdioxid oder Cerdioxid, den Oxiden von Zink, Eisen oder Chrom, Eisenblau, Ruß und den Calciumsalzen, Bariumsalzen, Aluminiumsalzen oder Zirconiumsalzen und den Salzen von säurefarbstoffen, wie Halogen-Säurefarbstoffen, Azofarbstoffen oder Anthrachinonfarbstoffen.

27. Zusammensetzung nach Anspruch 25, worin die Pigmente mit siliconhaltigen Verbindungen, wie PDMS und/oder Polymeren, insbesondere Polyethylenen, umhüllt sind.

28. Zusammensetzung nach einem der Ansprüche 14 bis 27, worin das wasserlösliche Färbemittel ausgewählt ist unter dem Dinatriumsalz von Ponceau, dem Dinatriumsalz von Alizaringrün, Chinolingelb, dem Trinatriumsalz von Amaranth, dem Dinatriumsalz von Tartrazin, dem Mononatriumsalz von Rhodamin, dem Dinatriumsalz von Fuchsin und Xanthophyll.

29. Zusammensetzung nach einem der Ansprüche 13 bis 28, die in Form einer Zusammensetzung zum Schminken der Haut, der Semi-Schleimhäute, der Schleimhäute und/oder der Hautanhangsgebilde, wie als Make-up, Wangenrouge, Lidschatten, Lippenstift, Mascara, Eyeliner oder Nagellack; einer Pflegegrundmasse für die Lippen; eines Produktes zur Pflege der Haut, der Schleimhäute, der Semi-Schleimhäute und/oder der Hautanhangsgebilde; eines hygienischen oder pharmazeutischen Produktes; eines Sonnenschutzproduktes oder eines selbstbräunenden Produktes oder eines Produktes zur Haarbehandlung vorliegt.

30. Make-up ohne Transfer, das mindestens ein gegebenenfalls vernetztes Copolymer enthält, das zum überwiegenden Teil aus Monomeren einer einfach olefinisch ungesättigten Carbonsäure mit 3 bis 6 Kohlenstoffatomen oder des Anhydrids dieser Säure und zu einem kleineren Teil aus Monomeren eines Acrylsäureesters mit Fettkette besteht.

31. Verfahren zum Abschwächen, vermindern und/oder Unterbinden des Transfers einer kosmetischen, dermatologischen, hygienischen und/oder pharmazeutischen Zusammensetzung, insbesondere einer Zusammensetzung zum Schminken und/oder zur Pflege der Haut, der Schleimhäute, der Semi-Schleimhäute und/oder der Hautanhangsgebilde, das darin besteht, in die Zusammensetzung mindestens ein gegebenenfalls vernetztes Copolymer einzubringen, das zum überwiegenden Teil aus Monomeren einer einfach olefinisch ungesättigten Carbonsäure mit 3 bis 6 Kohlenstoffatomen oder des Anhydrids dieser Säure und zu einem kleineren Teil aus Monomeren eines Acrylsäureesters mit Fettkette besteht.

32. Verfahren zum Schminken der Haut, der Semi-Schleimhäute, der Schleimhäute und/oder der Hautanhangsgebilde, das darin besteht, auf diese eine in einem der Ansprüche 13 bis 29 definierte Zusammensetzung oder ein in Anspruch 30 definiertes Make-up aufzutragen.
